# EUROPEAN PATENT SPECIFICATION

(11) **EP 1 073 435 B1**
(45) Date of publication and mention of the grant of the patent: **07.07.2004**
(21) Application number: 99920103.1
(22) Date of filing: 28.04.1999
(51) Int. Cl.: A61K 31/415, A61K 31/44, A61P 35/00, A61P 9/10, A61P 1/04

(54) **SUBSTITUTED TRICYCLIC PYRAZOLE DERIVATIVES WITH PROTEIN KINASE ACTIVITY**
SUBSTITUIERTE TRIZYKLISCHE PYRAZOLDERIVATE MIT PROTEIN KINASE AKTIVITÄT
DERIVES SUBSTITUES DE PYRAZOLE TRICYCLIQUE EXER ANT UNE ACTIVITE DE PROTEINE KINASE

(30) Priority: 30.04.1998 US 83648 P
(43) Date of publication of application: 07.02.2001
(73) Proprietor: Abbott GmbH & Co. KG, 65205 Wiesbaden (DE)
(72) Inventor: ARNOLD, Lee, Westborough, MA 01581 (US); RAFFERTY, Paul Knoll Pharmaceuticals, Nottingham NG1 1GF (GB); HOCKLEY, Michael Knoll Pharmaceuticals, Nottingham NG1 1GF (GB); TURNER, Allyson Knoll Pharmaceuticals, Nottingham NG1 1GF (GB)
(74) Representative: Kinzebach, Werner, Dr.
(86) International application number: PCT/US1999/009185
(87) International publication number: WO 1999/055335

(56) References cited:
- WO-A-94/10162
- WO-A-95/07893
- WO-A-95/19774
- WO-A-99/17769
- US-A- 3 816 437
- US-A- 3 816 438
- US-A- 3 843 664
- US-A- 3 843 666
- US-A- 3 932 430
- ZAYED, A. ET AL: "Reactions of 6,7-benzindazole-3-carboxylic acid hydrazide. Synthesis of some heterocycles of potential biological activity" EGYPT. J. CHEM. (1982), VOLUME DATE 1981, 24(4-6), 389-96 , XP002112689

## Description

### BACKGROUND OF THE INVENTION

There are at least 400 enzymes identified as protein kinases. These enzymes catalyze the phosphorylation of target protein substrates. The phosphorylation is usually a transfer reaction of a phosphate group from ATP to the protein substrate. The specific structure in the target substrate to which the phosphate is transferred is a tyrosine, serine or threonine residue. Since these amino acid residues are the target structures for the phosphoryl transfer, these protein kinase enzymes are commonly referred to as tyrosine kinases or serine/threonine kinases.

The phosphorylation reactions, and counteracting phosphatase reactions, at the tyrosine, serine and threonine residues are involved in countless cellular processes that underlie responses to diverse intracellular signals (typically mediated through cellular receptors), regulation of cellular functions, and activation or deactivation of cellular processes. A cascade of protein kinases often participate in intracellular signal transduction and are necessary for the realization of these cellular processes. Because of their ubiquity in these processes, the protein kinases can be found as an integral part of the plasma membrane or as cytoplasmic enzymes or localized in the nucleus, often as components of enzyme complexes. In many instances, these protein kinases are an essential element of enzyme and structural protein complexes that determine where and when a cellular process occurs within a cell.

### Protein Tyrosine Kinases.

Protein tyrosine kinases (PTKs) are enzymes which catalyse the phosphorylation of specific tyrosine residues in cellular proteins. This post-translational modification of these substrate proteins, often enzymes themselves, acts as a molecular switch regulating cell proliferation, activation or differentiation (for review, see Schlessinger and Ulrich, 1992, Neuron 9:383-391). Aberrant or excessive PTK activity has been observed in many disease states including benign and malignant proliferative disorders as well as diseases resulting from inappropriate activation of the immune system (e.g.. autoimmune disorders), allograft rejection, and graft vs. host disease. In addition, endothelial-cell specific receptor PTKs such as KDR and Tie-2 mediate the angiogenic process, and are thus involved in supporting the progression of cancers and other diseases involving inappropriate vascularization (e.g.. diabetic retinopathy, choroidal neovascularization due to age-related macular degeneration, psoriasis, arthritis, infantile hemangiomas).

Tyrosine kinases can be of the receptor-type (having extracellular, transmembrane and intracellular domains) or the non-receptor type (being wholly intracellular).

### Receptor Tyrosine Kinases (RTKs).

The RTKs comprise a large family of transmembrane receptors with diverse biological activities. At present, at least nineteen (19) distinct RTK subfamilies have been identified. The receptor tyrosine kinase (RTK) family includes receptors that are crucial for the growth and differentiation of a variety of cell types (Yarden and Ullrich, Ann. Rev. Biochem. 57:433-478, 1988; Ullrich and Schlessinger, Cell 61:243-254, 1990). The intrinsic function of RTKs is activated upon ligand binding, which results in phosphorylation of the receptor and multiple cellular substrates, and subsequently in a variety of cellular responses (Ullrich & Schlessinger, 1990, Cell 61:203-212). Thus, receptor tyrosine kinase mediated signal transduction is initiated by extracellular interaction with a specific growth factor (ligand), typically followed by receptor dimerization, stimulation of the intrinsic protein tyrosine kinase activity and receptor trans-phosphorylation. Binding sites are thereby created for intracellular signal transduction molecules and lead to the formation of complexes with a spectrum of cytoplasmic signaling molecules that facilitate the appropriate cellular response. (e.g.. cell division, differentiation, metabolic effects, changes in the extracellular microenvironment) (see Schlessinger and Ullrich, 1992, Neuron 9:1-20).

Proteins with SH2 (src homology -2) or phosphotyrosine binding (PTB) domains bind activated tyrosine kinase receptors and their substrates with high affinity to propagate signals into cells. Both of the domains recognize phosphotyrosine (Fantl et al., 1992, Cell 69:413-423; Songyang et al., 1994, Mol. Cell. Biol. 14:2777-2785; Songyang et al., 1993, Cell 72:767-778; Koch et al., 1991, Science 252:668-678; Shoelson, Curr. Opin. Chem. Biol. (1997), 1(2), 227-234; and Cowburn, Curr. Opin. Struct. Biol. (1997), 7(6), 835-838). Several intracellular substrate proteins that associate with receptor tyrosine kinases (RTKs) have been identified. They may be divided into two principal groups: (1) substrates which have a catalytic domain; and (2) substrates which lack such a domain but serve as adapters and associate with catalytically active molecules (Songyang et al., 1993, Cell 72:767-778). The specificity of the interactions between receptors or proteins and SH2 or PTB domains of their substrates is determined by the amino acid residues immediately surrounding the phosphorylated tyrosine residue. For example, differences in the binding affinities between SH2 domains and the amino acid sequences surrounding the phosphotyrosine residues on particular receptors correlate with the observed differences in their substrate phosphorylation profiles (Songyang et al., 1993, Cell 72:767-778). Observations suggest that the function of each receptor tyrosine kinase is determined not only by its pattern of expression and ligand availability but also by the array of downstream signal transduction pathways that are activated by a particular receptor as well as the timing and duration of those stimuli. Thus, phosphorylation provides an important regulatory step which determines the selectivity of signaling pathways recruited by specific growth factor receptors, as well as differentiation factor receptors.

Several receptor tyrosine kinases, and growth factors that bind thereto, have been suggested to play a role in angiogenesis, although some may promote angiogenesis indirectly (Mustonen and Alitalo, J. Cell Biol. 129:895-898, 1995). One such receptor tyrosine kinase, known as "fetal liver kinase 1" (FLK-1), is a member of the type III subclass of RTKs. An alternative designation for human FLK-1 is "kinase insert domain-containing receptor'' (KDR) (Terman et al., Oncogene 6:1677-83, 1991). Another alternative designation for FLK-1/KDR is "vascular endothelial cell growth factor receptor 2" (VEGFR-2) since it binds VEGF with high affinity. The murine version of FLK-1/VEGFR-2 has also been called NYK (Oelrichs et al, Oncogene 8(1):11-15, 1993). DNAs encoding mouse, rat and human FLK-1 have been isolated, and the nucleotide and encoded amino acid sequences reported (Matthews et al., Proc. Natl. Acad. Sci. USA, 88:9026-30, 1991; Terman et al., 1991, supra; Terman et al., Biochem. Biophys. Res. Comm. 187:1579-86, 1992; Sarzani et al., supra; and Millauer et al., Cell 72:835-846, 1993). Numerous studies such as those reported in Millauer et al., supra. suggest that VEGF and FLK-1/KDR/VEGFR-2 are a ligand-receptor pair that play an important role in the proliferation of vascular endothelial cells, and formation and sprouting of blood vessels, termed vasculogenesis and angiogenesis, respectively.

Another type III subclass RTK designated "fms-like tyrosine kinase-1" (Flt-1) is related to FLK-1/KDR (DeVries et al. Science 255;989-991, 1992; Shibuya et al., Oncogene 5:519-524, 1990). An alternative designation for Flt-1 is "vascular endothelial cell growth factor receptor 1" (VEGFR-1). To date, members of the FLK-1/KDR/VEGFR-2 and Flt-1/ VEGFR-1 subfamilies have been found expressed primarily on endothelial cells. These subclass members are specifically stimulated by members of the vascular endothelial cell growth factor (VEGF) family of ligands (Klagsburn and D'Amore, Cytokine & Growth Factor Reviews 7: 259-270, 1996). Vascular endothelial cell growth factor (VEGF) binds to Flt-1 with higher affinity than to FLK-1/KDR and is mitogenic toward vascular endothelial cells (Terman et al.. 1992, supra; Mustonen et al. supra; DeVries et al., supra). Flt-1 is believed to be essential for endothelial organization during vascular development. Flt-1 expression is associated with early vascular development in mouse embryos, and with neovascularization during wound healing (Mustonen and Alitalo, supra). Expression of Flt-1 in adult organs such as kidney glomeruli suggests an additional function for this receptor that is not related to cell growth (Mustonen and Alitalo, supra).

As previously stated, recent evidence suggests that VEGF plays a role in the stimulation of both normal and pathological angiogenesis (Jakeman et al., Endocrinology 133: 848-859, 1993; Kolch et al., Breast Cancer Research and Treatment 36: 139-155, 1995; Ferrara et al., Endocrine Reviews 18(1); 4-25, 1997; Ferrara et al., Regulation of Angiogenesis (ed. L. D. Goldberg and E.M. Rosen), 209-232, 1997). In addition, VEGF has been implicated in the control and enhancement of vascular permeability (Connolly, et al., J. Biol. Chem. 264: 20017-20024, 1989; Brown et al., Regulation of Angiogenesis (ed. L.D. Goldberg and E.M. Rosen). 233-269, 1997).

Different forms of VEGF arising from alternative splicing of mRNA have been reported, including the four species described by Ferrara et al. (.J. Cell. Biochem. 47:211-218, 1991). Both secreted and predominantly cell-associated species of VEGF have been identified by Ferrara et al. supra, and the protein is known to exist in the form of disulfide linked dimers.

Several related homologs of VEGF have recently been identified. However, their roles in normal physiological and disease processes have not yet been elucidated. In addition, the members of the VEGF family are often coexpressed with VEGF in a number of tissues and are, in general, capable of forming heterodimers with VEGF. This property likely alters the receptor specificity and biological effects of the heterodimers and further complicates the elucidation of their specific functions as illustrated below (Korpelainen and Alitalo, Curr. Opin. Cell Biol., 159-164, 1998 and references cited therein).

Placenta growth factor (PlGF) has an amino acid sequence that exhibits significant homology to the VEGF sequence (Park et al., J. Biol. Chem. 269:25646-54, 1994; Maglione et al. Oncogene 8:925-31, 1993). As with VEGF, different species of PlGF arise from alternative splicing of mRNA, and the protein exists in dimeric form (Park et al., supra). PIGF-1 and PIGF-2 bind to Flt-1 with high affinity, and PlGF-2 also avidly binds to neuropilin-1 (Migdal et al,J. Biol. Chem. 273 (35): 22272-22278), but neither binds to FLK-1/KDR (Park et al., supra). PlGF has been reported to potentiate both the vascular permeability and mitogenic effect of VEGF on endothelial cells when VEGF is present at low concentrations (purportedly due to heterodimer formation) (Park et al., supra).

VEGF-B is produced as two isoforms (167 and 185 residues) that also appear to bind Flt-1/VEGFR-1. It may play a role in the regulation of extracellular matrix degradation, cell adhesion, and migration through modulation of the expression and activity of urokinase type plasminogen activator and plasminogen activator inhibitor 1 (Pepper et al, Proc. Natl. Acad. Sci. U. S. A. (1998), 95(20): 11709-11714).

VEGF-C was originally cloned as a ligand for VEGFR-3/Flt-4 which is primarily expressed by lymphatic endothelial cells. In its fully processed form, VEGF-C can also bind KDR/VEGFR-2 and stimulate proliferation and migration of endothelial cells in vitro and angiogenesis in in vivo models ( Lymboussaki et al, Am. J. Pathol. (1998), 153(2): 395-403; Witzenbichler et al, Am. J. Pathol. (1998), 153(2), 381-394). The transgenic overexpression of VEGF-C causes proliferation and enlargement of only lymphatic vessels, while blood vessels are unaffected. Unlike VEGF, the expression of VEGF-C is not induced by hypoxia (Ristimaki et al, J. Biol. Chem. (1998), 273(14),8413-8418).

The most recently discovered VEGF-D is structurally very similar to VEGF-C. VEGF-D is reported to bind and activate at least two VEGFRs. VEGFR-3/Flt-4 and KDR/VEGFR-2. It was originally cloned as a c-fos inducible mitogen for fibroblasts and is most prominently expressed in the mesenchymal cells of the lung and skin (Achen et al, Proc. Natl. Acad. Sci. U. S. A. (1998), 95(2), 548-553 and references therein).

VEGF-C and VEGF-D have been claimed to induce increases in vascular permeability in vivo in a Miles assay when injected into cutaneous tissue (PCT/US97/14696; WO98/07832, Witzenbichler et al., supra). The physiological role and significance of these ligands in modulating vascular hyperpermeability and endothelial responses in tissues where they are expressed remains uncertain.

Based upon emerging discoveries of other homologs of VEGF and VEGFRs and the precedents for ligand and receptor heterodimerization, the actions of such VEGF homologs may involve formation of VEGF ligand heterodimers, and/or heterodimerization of receptors, or binding to a yet undiscovered VEGFR (Witzenbichler et al., supra). Also, recent reports suggest neuropilin-1 (Migdal et al, supra) or VEGFR-3/Flt-4 (Witzenbichler et al., supra), or receptors other than KDR/VEGFR-2 may be responsible for the induction of vascular permeability (Stacker, S.A., Vitali, A., Domagala, T., Nice, E., and Wilks, A.F., "Angiogenesis and Cancer" Conference, Amer. Assoc. Cancer Res., Jan. 1998, Orlando, FL; Williams, Diabetelogia 40: S118-120 (1997)). Until now, no direct evidence for the essential role of KDR in VEGF-mediated vascular hyperpermeability has been disclosed.

### The Non-Receptor Tyrosine Kinases.

The non-receptor tyrosine kinases represent a collection of cellular enzymes which lack extracellular and transmembrane sequences. At present, over twenty-four individual non-receptor tyrosine kinases, comprising eleven (11) subfamilies (Src. Frk. Btk. Csk, Abl. Zap70, Fes/Fps, Fak, Jak, Ack and LIMK) have been identified. At present, the Src subfamily of non-receptor tyrosine kinases is comprised of the largest number of PTKs and include Src, Yes, Fyn, Lyn, Lck, Blk, Hck, Fgr and Yrk. The Src subfamily of enzymes has been linked to oncogenesis. A more detailed discussion of non-receptor tyrosine kinases is provided in Bolen, 1993, Oncogene 8:2025-2031, which is incorporated herein by reference.

Many of the tyrosine kinases, whether an RTK or non-receptor tyrosine kinase, have been found to be involved in cellular signaling pathways involved in numerous pathogenic conditions, including cancer, psoriasis, and other hyperproliferative disorders or hyper-immune responses.

### Development of Compounds to Modulate the PTKs.

In view of the surmised importance of PTKs to the control, regulation, and modulation of cell proliferation, the diseases and disorders associated with abnormal cell proliferation, many attempts have been made to identify receptor and non-receptor tyrosine kinase "inhibitors" using a variety of approaches, including the use of mutant ligands (U.S. Patent No. 4,966,849), soluble receptors and antibodies (Application No. WO 94/10202; Kendall & Thomas, 1994, Proc. Natl. Acad. Sci 90:10705-09; Kim et al., 1993, Nature 362:841-844), RNA ligands (Jellinek, et al., Biochemistry 33:10450-56; Takano, et al., 1993, Mol. Bio. Cell 4:358A; Kinsella, et al. 1992, Exp. Cell Res. 199:56-62; Wright, et al., 1992, J. Cellular Phys. 152:448-57) and tyrosine kinase inhibitors (WO 94/03427; WO 92/21660; WO 91/15495; WO 94/14808; U.S. Patent No. 5,330,992; Mariani, et al., 1994, Proc. Am. Assoc. Cancer Res. 35:2268).

More recently, attempts have been made to identify small molecules which act as tyrosine kinase inhibitors. For example, bis monocyclic, bicyclic or heterocyclic aryl compounds (PCT WO 92/20642) and vinylene-azaindole derivatives (PCT WO 94/14808) have been described generally as tyrosine kinase inhibitors. Styryl compounds (U.S. Patent No. 5,217,999), styryl-substituted pyridyl compounds (U.S. Patent No. 5,302,606), certain quinazoline derivatives (EP Application No. 0 566 266 Al; Expert Opin. Ther. Pat. (1998), 8(4): 475-478), selenoindoles and selenides (PCT WO 94/03427), tricyclic polyhydroxylic compounds (PCT WO 92/21660) and benzylphosphonic acid compounds (PCT WO 91/15495) have been described as compounds for use as tyrosine kinase inhibitors for use in the treatment of cancer. Anilinocinnolines (PCT WO97/34876) and quinazoline derivative compounds (PCT WO97/22596; PCT WO97/42187) have been described as inhibitors of angiogenesis and vascular permeability.

In addition, attempts have been made to identify small molecules which act as serine/threonine kinase inhibitors. In particular, bis(indolylmaleimide) compounds have been described as inhibiting particular PKC serine/threonine kinase isoforms whose dysfunction is associated with altered vascular permeability in VEGF-related diseases (PCT WO97/40830; PCT WO97/40831).

The identification of effective small compounds which specifically inhibit signal transduction by modulating the activity of receptor and non-receptor tyrosine and serine/threonine kinases to regulate and modulate abnormal or inappropriate cell proliferation. differentiation, or metabolism is therefore desirable. In particular, the identification of methods and compounds that specifically inhibit the function of a tyrosine kinase which is essential for angiogenic processes or the formation of vascular hyperpermeability leading to edema, ascites, effusions, exudates, and macromolecular extravasation and matrix deposition as well as associated disorders would be beneficial.

### SUMMARY OF THE INVENTION

This invention is directed to compounds represented by either of the formulas: 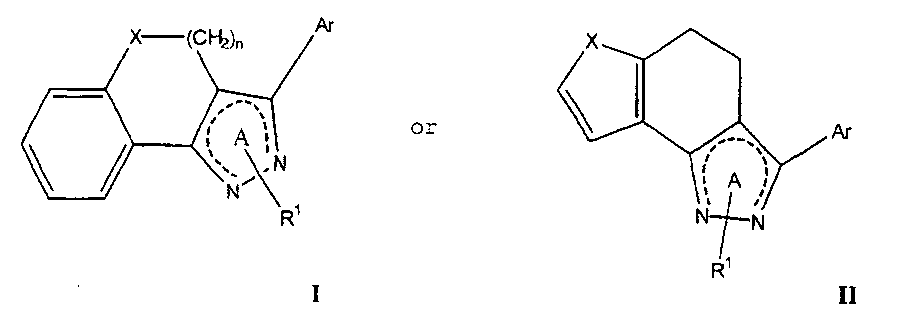 wherein:
X is oxygen or sulfur;
ring A represents the structures: 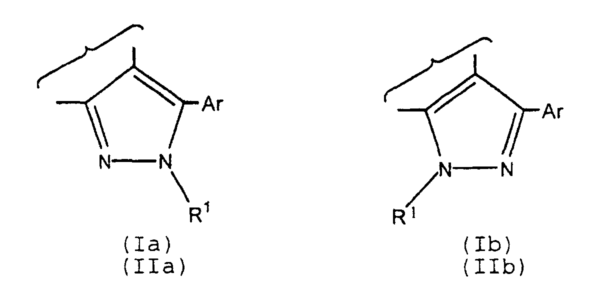
n is 1 or 2;
AR is 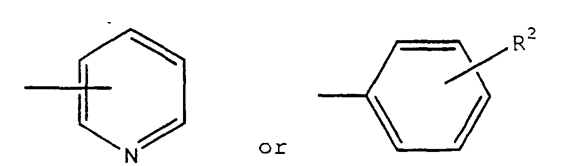 and
   R¹ is hydrogen, methyl, COR³, or SO₂R³;
   R² represents a halogen having an atomic weight of about 19 to 36; and
   R³ is -CH₃ or 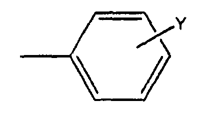 wherein Y is hydrogen, chlorine or -CH₃.

Stereoisomers and mixtures of stereoisomers are included in this invention. Tautomers of the compounds are also within the scope of the invention. Pharmaceutically acceptable acid addition salts of these compounds are also included in this invention.

The compounds of this invention are useful as inhibitors of protein kinase activity. In particular, the compounds of this invention are useful as inhibitors of protein kinases that are important in the processes of angiogenesis and/or vascular permeability. Since these compounds are anti-angiogenic and/or antiedematous, they are important substances for inhibiting the progression of disease states where angiogenesis and/or edema are manifested.

A preferred compound of the present invention has the formula:

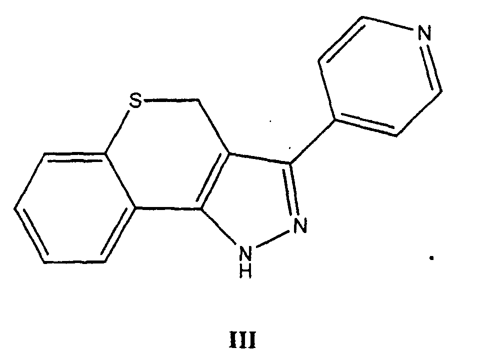

The compounds of this invention are useful as inhibitors of serine/threonine and tyrosine kinases. In particular, the compounds of this invention are useful as inhibitors of tyrosine kinases that are important in hyperproliferative diseases, especially in the process of angiogenesis. Since these compounds are anti-angiogenic, they are important substances for inhibiting the progression of disease states where angiogenesis is an important component.

The present invention further includes the use of these compounds in pharmaceutical compositions with a pharmaceutically effective amount of the above-described compounds and a pharmaceutically acceptable carrier or excipient. These pharmaceutical compositions can be administered to individuals to slow or halt the process of angiogenesis in angiogenesis-aided diseases or to treat edema effusions, exudates, or ascites and other conditions associated with vascular hyperpermeability.

### DETAILED DESCRIPTION OF THE INVENTION

The compounds of this invention have antiangiogenic properties. These antiangiogenic properties are due at least in part to the inhibition of protein tyrosine kinases essential for angiogenic processes. For this reason, these compounds can be used as active agents against such disease states as arthritis, atherosclerosis, psoriasis, hemangiomas, myocardial angiogenesis, coronary and cerebral collaterals, ischemic limb angiogenesis, wound healing, peptic ulcer *Helicobacter* related diseases, fractures, cat scratch fever, rubeosis, neovascular glaucoma and retinopathies such as those associated with diabetic retinopathy or age-related macular degeneration. In addition, some of these compounds can be used as active agents against solid tumors, malignant ascites, hematopoietic cancers and hyperproliferative disorders such as thyroid hyperplasia (especially Grave's disease), and cysts (such as hypervascularity of ovarian stroma characteristic of polycystic ovarian syndrome (Stein-Leventhal syndrome)) since such diseases require a proliferation of blood vessel cells for growth and/or metastasis.

Further, some of these compounds can be used as active agents against bums, chronic lung disease, stroke, polyps, anaphylaxis, chronic and allergic inflammation, ovarian hyperstimulation syndrome, brain tumor-associated cerebral edema, high-altitude, trauma or hypoxia induced cerebral or pulmonary edema, ocular and macular edema, ascites, and other diseases where vascular hyperpermeability, effusions, exudates, protein extravasation, or edema is a manifestation of the disease. The compounds will also be useful in treating disorders in which protein extravasation leads to the deposition of fibrin and extracellular matrix, promoting stromal proliferation (e.g. fibrosis, cirrhosis and carpal tunnel syndrome).

VEGF's are unique in that they are the only angiogenic growth factors known to contribute to vascular hyperpermeability and the formation of edema. Indeed, vascular hyperpermeability and edema that is associated with the expression or administration of many other growth factors appears to be mediated via VEGF production. Inflammatory cytokines stimulate VEGF production. Hypoxia results in a marked upregulation of VEGF in numerous tissues, hence situations involving infarct, occlusion, ischemia, anemia, or circulatory impairment typically invoke VEGF/VPF mediated responses. Vascular hyperpermeability, associated edema, altered transendothelial exchange and macromolecular extravasation, which is often accompanied by diapedesis, can result in excessive matrix deposition, aberrant stromal proliferation, fibrosis, etc. Hence, VEGF-mediated hyperpermeability can significantly contribute to disorders with these etiologic features.

It is envisaged that the disorders listed above are mediated to a significant extent by protein tyrosine kinase activity involving the KDR/VEGFR-2 and/or the Flt-1/VEGFR-1 tyrosine kinases. By inhibiting the activity of these tyrosine kinases, the progression of the listed disorders is inhibited because the angiogenic or vascular hyperpermeability component of the disease state is severely curtailed. The action of the compounds of this invention, by their selectivity for specific tyrosine kinases. result in a minimization of side effects that would occur if less selective tyrosine kinase inhibitors were used.

The compounds of this invention have inhibitory activity against protein kinases. That is, these compounds modulate signal transduction by protein kinases. Compounds of this invention inhibit protein kinases from serine/threonine and tyrosine kinase classes. In particular, these compounds selectively inhibit the activity of the KDR/FLK-1/VEGFR-2 tyrosine kinases. Certain compounds of this invention also inhibit the activity of additional tyrosine kinases such as Flt-1/VEGFR-1. Src-subfamily kinases such as Lck, Src, fyn, yes, etc. Additionally, some compounds of this invention significantly inhibit serine/threonine kinases such as CDKs which play an essential role in cell-cycle progression. The potency and specificity of the generic compounds of this invention towards a particular protein kinase can often be altered and optimized by variations in the nature, number and arrangement of the substituents (i.e., R¹, R² and R³) of these compounds and conformational restrictions. In addition the metabolites of certain compounds may also possess significant protein kinase inhibitory activity.

The compounds of this invention, when administered to individuals in need of such compounds, inhibit vascular hyperpermeability and the formation of edema in these individuals. These compounds act, it is believed, by inhibiting the activity of KDR tyrosine kinase which is involved in the process of vascular hyperpermeability and edema formation. The KDR tyrosine kinase may also be referred to as FLK-1 tyrosine kinase, NYK tyrosine kinase or VEGFR-2 tyrosine kinase. KDR tyrosine kinase is activated when vascular endothelial cell growth factor (VEGF) or another activating ligand (such as VEGF-C, VEGF-D or HIV Tat protein) binds to a KDR tyrosine kinase receptor which lies on the surface of vascular endothelial cells. Following such KDR tyrosine kinase activation, hyperpermeability of the blood vessels occurs and fluid moves from the blood stream past the blood vessel walls into the interstitial spaces, thereby forming an area of edema. Diapedesis also often accompanies this response. Similarly, excessive vascular hyperpermeability can disrupt normal molecular exchange across the endothelium in critical tissues and organs (e.g., lung and kidney), thereby causing macromolecular extravasation and deposition. Following this acute response to KDR stimulation which is believed to facilitate the subsequent angiogenic process, prolonged KDR tyrosine kinase stimulation results in the proliferation and chemotaxis of vascular endothelial cells and formation of new vessels. By inhibiting KDR tyrosine kinase activity, either by blocking the production of the activating ligand, by blocking the activating ligand binding to the KDR tyrosine kinase receptor, by preventing receptor dimerization and transphosphorylation. by inhibiting the enzyme activity of the KDR tyrosine kinase (inhibiting the phosphorylation function of the enzyme) or by some other mechanism that interrupts its downstream signaling (D. Mukhopedhyay *et al.,* *Cancer Res. 58*:1278-1284 (1998) and references therein), hyperpermeability, as well as associated extravasation, subsequent edema formation and matrix deposition, and angiogenic responses, may be inhibited and minimized.

One group of preferred compounds of this invention has the property of inhibiting KDR tyrosine kinase activity without significantly inhibiting Flt-1 tyrosine kinase activity (Flt-1 tyrosine kinase is also referred to as VEGFR-1 tyrosine kinase). Both KDR tyrosine kinase and Flt-1 tyrosine kinase are activated by VEGF binding to KDR tyrosine kinase receptors and to Flt-1 tyrosine kinase receptors, respectively. Since Flt-1 tyrosine kinase activity may mediate important events in endothelial maintenance and vascular function, an inhibition of this enzyme activity may lead to toxic or adverse effects. At the very least, such inhibition is unnecessary for blocking the angiogenic responses, induction of vascular hyperpermeability and the formation of edema, so it is wasteful and of no value to the individual. Certain preferred compounds of this invention are unique because they inhibit the activity of one VEGF-receptor tyrosine kinase (KDR) that is activated by activating ligands but do not inhibit other receptor tyrosine kinases, such as Flt-1, that are also activated by certain activating ligands. The preferred compounds of this invention are, therefore, selective in their tyrosine kinase inhibitory activity.

The compounds of the present invention are also useful in the treatment of ulcers - bacterial, fungal, Mooren ulcers and ulcerative colitis.

The compounds of the present invention are also useful in the treatment of conditions wherein undesired angiogenesis, edema, or stromal deposition occurs in viral infections such as Herpes simplex, Herpes Zoster, AIDS, Kaposi's sarcoma, protozoan infections and toxoplasmosis, following trauma, radiation, stroke, endometriosis, ovarian hyperstimulation syndrome, systemic lupus, sarcoidosis, synovitis, Crohn's disease, sickle cell anaemia, Lyme's disease, pemphigoid, Paget's disease, hyperviscosity syndrome, Osler-Weber-Rendu disease, chronic inflammation, chronic occlusive pulmonary disease, asthma, rheumatoid arthritis and osteoarthritis.

The compounds of the present invention are also useful in the treatment of ocular conditions such as ocular and macular edema, ocular neovascular disease, scleritis, radial keratotomy, uveitis, vitritis, myopia, optic pits, chronic retinal detachment, post-laser complications, conjunctivitis, Stargardt's disease and Eales disease in addition to retinopathy and macular degeneration.

The compounds of the present invention are also useful in the treatment of cardiovascular conditions such as atherosclerosis, restenosis, vascular occlusion and carotid obstructive disease.

The compounds of the present invention are also useful in the treatment of cancer related indications such as solid tumors, sarcomas (especially Ewing's sarcoma and osteosarcoma), retinoblastoma, rhabdomyosarcomas, neuroblastoma, hematopoietic malignancies, including leukaemia and lymphoma, tumor-induced pleural or pericardial effusions, and malignant ascites.

The compounds of the present invention are also useful in the treatment of diabetic conditions such as glaucoma, diabetic retinopathy and microangiopathy.

It is envisaged that the disorders listed above are mediated to a significant extent by protein tyrosine kinase activity involving the VEGF receptors (e.g. KDR and Flt-1). By inhibiting the activity of these receptor tyrosine kinases, the progression of the listed disorders is inhibited because the angiogenic component of the disease state is severely curtailed. The action of the compounds of this invention, by their selectivity for specific tyrosine kinases, result in a minimization of side effects that would occur if less selective tyrosine kinase inhibitors were used.

In another aspect the present invention provides compounds of formula I as defined initially above (including the provisos) for use as medicaments, particularly as inhibitors of protein kinase activity for example tyrosine kinase activity, serine kinase activity and threonine kinase activity. In yet another aspect the present invention provides the use of compounds of formula I as defined initially above (including the provisos) in the manufacture of a medicament for use in the inhibition of protein kinase activity.

In this invention, the following definitions are applicable:
"Pharmaceutically acceptable salts" refers to those salts which retain the biological effectiveness and properties of the free bases and which are obtained by reaction with inorganic acids such as hydrochloric acid, hydrobromic acid, sulfuric acid, nitric acid, phosphoric acid or organic acids such as sulfonic acid, carboxylic acid, organic phosphoric acid, methanesulfonic acid, ethanesulfonic acid, p-toluenesulfonic acid, salicylic acid, lactic acid, tartaric acid and the like.
"Alkyl" refers to a saturated aliphatic hydrocarbon, including straight-chain and branched-chain groups having 1 to 4 carbons.
"Alkoxy" refers to an "O-alkyl" group, where "alkyl" is defined as described above.

### Pharmaceutical Formulations

The identified compounds of this invention can be administered to a human patient by themselves or in pharmaceutical compositions where they are mixed with suitable carriers or excipient(s) at doses to treat or ameliorate a variety of disorders. Mixtures of these compounds can also be administered to the patient as a simple mixture or in suitable formulated pharmaceutical compositions. A therapeutically effective dose further refers to that amount of the compound or compounds sufficient to result in the prevention of disease progression. Techniques for formulation and administration of the compounds of the instant application may be found in "Remington's Pharmaceutical Sciences," Mack Publishing Co., Easton, PA, latest edition.

### Routes of Administration

Suitable routes of administration may, for example, include oral, eyedrop, rectal, transmucosal. topical, or intestinal administration; parenteral delivery, including intramuscular, subcutaneous, intramedullary injections, as well as intrathecal, direct intraventricular, intravenous, intraperitoneal, intranasal, or intraocular injections.

Alternately, one may administer the compound in a local rather than a systemic manner, for example, via injection of the compound directly into a solid tumor, often in a depot or sustained release formulation.

Furthermore, one may administer the drug in a targeted drug delivery system, for example, in a liposome coated with tumor-specific antibody. The liposomes will be targeted to and taken up selectively by the tumor.

### Composition/Formulation

The pharmaceutical compositions of the present invention may be manufactured in a manner that is itself known, e.g., by means of conventional mixing. dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries which facilitate processing of the active compounds into preparations which can be used pharmaceutically. Proper formulation is dependent upon the route of administration chosen.

For injection, the agents of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by combining the active compound with a solid excipient, optionally grinding a resulting mixture, and processing the mixture of granules, after adding suitable auxiliaries, if desired, to obtain tablets or dragee cores. Suitable excipients are, in particular, fillers such as sugars. including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide. lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may be formulated for parenteral administration by injection, e.g. bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly or by intramuscular injection). Thus, for example, the compounds may be formulated with suitable polymeric or hydrophobic materials (for example as an emulsion in an acceptable oil) or ion exchange resins, or as sparingly soluble derivatives, for example. as a sparingly soluble salt.

A pharmaceutical carrier for the hydrophobic compounds of the invention is a cosolvent system comprising benzyl alcohol, a nonpolar surfactant, a water-miscible organic polymer, and an aqueous phase. The cosolvent system may be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system dissolves hydrophobic compounds well, and itself produces low toxicity upon systemic administration. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of polysorbate 80; the fraction size of polyethylene glycol may be varied; other biocompatible polymers may replace polyethylene glycol, e.g. polyvinyl pyrrolidone; and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. Certain organic solvents such as dimethysulfoxide also may be employed, although usually at the cost of greater toxicity. Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Many of the protein kinase modulating compounds of the invention may be provided as salts with pharmaceutically compatible counterions. Pharmaceutically compatible salts may be formed with many acids, including but not limited to hydrochloric, sulfuric, acetic, lactic, tartaric, malic, succinic, etc. Salts tend to be more soluble in aqueous or other protonic solvents that are the corresponding free base forms.

### Effective Dosage

Pharmaceutical compositions suitable for use in the present invention include compositions wherein the active ingredients are contained in an effective amount to achieve its intended purpose. More specifically, a therapeutically effective amount means an amount effective to prevent development of or to alleviate the existing symptoms of the subject being treated. Determination of the effective amounts is well within the capability of those skilled in the art, especially in light of the detailed disclosure provided herein.

For any compound used in the method of the invention, the therapeutically effective dose can be estimated initially from cellular assays. For example, a dose can be formulated in animal models to achieve a circulating concentration range that includes the IC₅₀ as determined in cellular assays (i.e., the concentration of the test compound which achieves a half-maximal inhibition of the PTK activity). Such information can be used to more accurately determine useful doses in humans.

A therapeutically effective dose refers to that amount of the compound that results in amelioration of symptoms or a prolongation of survival in a patient. Toxicity and therapeutic efficacy of such compounds can be determined by standard pharmaceutical procedures in cell cultures or experimental animals, e.g., for determining the maximum tolerated dose (MTD) and the ED₅₀ (the dose therapeutically effective in 50% of the population). The dose ratio between toxic and therapeutic effects is the therapeutic index and it can be expressed as the ratio between MTD and ED₅₀. Compounds which exhibit high therapeutic indices are preferred. The data obtained from these cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient s condition. (See e.g. Fingl *et al.,* 1975. in "The Pharmacological Basis of Therapeutics", Ch. 1 p1).

Dosage amount and interval may be adjusted individually to provide plasma levels of the active moiety which are sufficient to maintain the kinase modulating effects. or minimal effective concentration (MEC). The MEC will vary for each compound but can be estimated from *in vitro* data; e.g. the concentration necessary to achieve 50-90% inhibition of the kinase using the assays described herein. Dosages necessary to achieve the MEC will depend on individual characteristics and route of administration. However, HPLC assays or bioassays can be used to determine plasma concentrations.

Dosage intervals can also be determined using the MEC value. Compounds should be administered using a regimen which maintains plasma levels above the MEC for 10-90% of the time, preferably between 30-90% and most preferably between 50-90%. In cases of local administration or selective uptake, the effective local concentration of the drug may not be related to plasma concentration.

The amount of composition administered will, of course, be dependent on the subject being treated, on the subject s weight, the severity of the affliction, the manner of administration and the judgment of the prescribing physician.

### Packaging

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. Compositions comprising a compound of the invention formulated in a compatible pharmaceutical carrier may also be prepared, placed in an appropriate container, and labeled for treatment of an indicated condition. Suitable conditions indicated on the label may include treatment of a cellular hyperproliferative disorder, inhibition of angiogenesis, treatment of fibrosis, diabetes, and the like.

In some formulations it may be beneficial to use the compounds of the present invention in the form of particles of very small size, for example as obtained by fluid energy milling.

In the compositions of the present invention the active compound may, if desired, be associated with other compatible pharmacologically active ingredients. For example, the compounds of this invention can be administered in combination with one or more additional pharmaceutical agents that inhibit or prevent the production of VEGF, attenuate intracellular responses to VEGF, block intracellular signal transduction, inhibit vascular hyperpermeability, reduce inflammation, or inhibit or prevent the formation of edema or neovascularization. The compounds of the invention can be administered prior to, subsequent to or simultaneously with the additional pharmaceutical agent, whichever course of administration is appropriate. The additional pharmaceutical agents include but are not limited to anti-edemic steroids, NSAIDS, ras inhibitors, anti-TNF agents, anti-IL 1 agents, antihistamines, PAF-antagonists, COX-1 inhibitors, COX-2 inhibitors, NO synthase inhibitors, PKC inhibitors and P13 kinase inhibitors. The compounds of the invention and the additional pharmaceutical agents act either additively or synergistically. Thus, the administration of such a combination of substances that inhibit vascular hyperpermeability and/or inhibit the formation of edema can provide greater relief from the deletrious effects of a hyperproliferative disorder, angiogenesis, vascular hyperpermeability or edema than the administration of either substance alone. In the treatment of malignant disorders combinations with antiproliferative or cytotoxic chemotherapies or radiation are anticipated.

The present invention also comprises the use of a compound of formula I as a medicament.

Both the Src and Syk families of kinases play pivotal roles in the regulation of immune function. The Src family currently includes Fyn, Lck, Fgr, Fes, Lyn, Src, Yes. Hck, and Blk. The Syk family is currently understood to include only Zap and Syk. The Janus family of kinases is involved in the transduction of growth factor and proinflammatory cytokine signals through a number of receptors. Although BTK and ITK. members of the Tec family ofkinases, play a less well understood role in immunobiology, their modulation by an inhibitor may prove therapeutically beneficial. The kinases RIP, IRAK-1, IRAK-2, NIK, IKK-1 and IKK-2 are involved in the signal transduction pathways for the key pro-inflammatory cytokines TNF and IL-1. By virtue of their ability to inhibit one or more of these kinases, compounds of formula I may function as immunomodulatory agents useful for the maintenance of allografts and the treatment of autoimmune disorders. Through their ability to regulate T cell activation or the potentiation of an inflammatory process, these compounds could be used to treat such autoimmune diseases. Transplants due to rejection phenomena, either host versus graft for solid organs or graft versus host for bone marrow, are limited by the toxicity of currently available immunosuppressive agents and would benefit from an efficacious drug with improved therapeutic index. Gene targeting experiments have demonstrated the essential role of Src in the biology of osteoclasts, the cells responsible for bone resorption. Compounds of formula I, through their ability to regulate Src, may also be useful in the treatment of osteoporosis, osteopetrosis, Paget's disease, tumor-induced hypercalcemia and in the treatment of bone metastases.

A number of protein kinases have been demonstrated to be protooncogenes. Chromosome breakage (at the ltk kinase break point on chromosome 5), translocation as in the case of the Abl gene with BCR (Philadelphia chromosome), truncation in instances such as c-Kit or EGFR, or mutation (e.g., Met) result in the creation of dysregulated proteins converting them from protooncogene to oncogene products. In other tumors, oncogenesis is driven by an autocrine or paracrine ligand/growth factor receptor interactions. Members of the src-family kinases are typically involved in downstream signal transduction thereby potentiating the oncogenesis and themselves may become oncogenic by over-expression or mutation. By inhibiting the protein kinase activity of these proteins the disease process may be disrupted. Vascular restenosis may involve process of FGF and/or PDGF - promoted smooth muscle and endothelial cell proliferation. Inhibition of FGFr or PDGFr kinase activity may be an efficacious strategy for inhibiting this phenomenon. Thus compounds of formula I which inhibit the kinase activity of normal or aberrant c-kit, c-met, c-fms, src-family members, EGFr, erbB2, erbB4, BCR-Abl, PDGFr, FGFr, and other receptor or cytosolic tyrosine kinases may be of value in the treatment of benign and neoplastic proliferative diseases.

In many pathological conditions (for example, solid primary tumors and metastases, Kaposi's sarcoma, rheumatoid arthritis, blindness due to inappropriate ocular neovascularization, psoriasis and atherosclerosis) disease progression is contingent upon persistent angiogenesis. Polypeptide growth factors often produced by the disease tissue or associated inflammatory cells, and their corresponding endothelial cell specific receptor tyrosine kinases (e.g., KDR/VEGFR-2, Flt-1/VEGFR-1, Tie-2/Tek and Tie) are essential for the stimulation of endothelial cell growth, migration, organization, differentiation and the establishment of the requisite new functional vasculature.

As a result of the "vascular permeability factor" activity of VEGF in mediating vascular hyperpermeability, VEGF-stimulation of a VEGFR kinase is also believed to play an important role in the formation of tumor ascites, cerebral and pulmonary edema, pleural and pericardial effusions, delayed-type hypersensitivity. reactions, tissue edema and organ dysfunction following trauma, bums, ischemia, diabetic complications, endometriosis, adult respiratory distress syndrome (ARDS), post-cardiopulmonary bypass-related hypotension and hyperpermeability, and ocular edema leading to glaucoma or blindness due to inappropriate neovascularization. In addition to VEGF, recently identified VEGF-C and VEGF-D, and HIV-Tat protein can also cause a vascular hyperpermeability response through the stimulation of a VEGFR kinase.

Tie-2 is expressed also in a select population of hematopoietic stem cells in which it may play a role in their recruitment, adhesion, regulation and differentiation *(Blood* **89**, 4317-4326 (1997)); this Tie-2 expressing population may serve as circulating angiogenic endothelial progenitors. Certain agents according to formula I capable of blocking the kinase activity of endothelial cell specific kinases could therefore inhibit disease progression involving these situations.

The compounds of formula I or a salt thereof or pharmaceutical compositions containing a therapeutically effective amount thereof may be used in the treatment of benign and neoplastic proliferative diseases and disorders of the immune system. Such diseases include autoimmune diseases, such as rheumatoid arthritis, thyroiditis, type 1 diabetes, multiple sclerosis, sarcoidosis, inflammatory bowel disease, myasthenia gravis and systemic lupus erythematosus; psoriasis, organ transplant rejection (eg. kidney rejection, graft versus host disease), benign and neoplastic proliferative diseases, human cancers such as lung, breast, stomach, bladder, colon, pancreas, ovarian, prostate and rectal cancer and hematopoietic cells (leukemia and lymphoma), and diseases involving inappropriate vascularization for example diabetic retinopathy, choroidal neovascularization due to age-related macular degeneration, and infantile hemangiomas in human beings. In addition, such inhibitors may be useful in the treatment of disorders involving VEGF mediated edema. ascites, effusions, and exudates, including for example macular edema, cerebral edema, and adult respiratory distress syndrome (ARDS).

The compounds of the present invention may also be useful in the prophylaxis of the above diseases.

A further aspect of the present invention provides the use of a compound of formula I or a salt thereof in the manufacture of a medicament for treating vascular hyperpermeability, angiogenesis-dependent disorders, proliferative diseases and/or disorders of the immune system in mammals, particularly human beings.

The present invention also provides a method of treating vascular hyperpermeability, inappropriate neovascularization, proliferative diseases and/or disorders of the immune system which comprises the administration of a therapeutically effective amount of a compound of formula I to a mammal, particularly a human being, in need thereof.

The in vitro potency of compounds in inhibiting these protein kinases may be determined by the procedures detailed below.

The potency of compounds can be determined by the amount of inhibition of the phosphorylation of an exogenous substrate (e.g., synthetic peptide (Z. Songyang et al., Nature. 373:536-539) by a test compound relative to control.

### EXEMPLIFICATIONS

### I. Synthesis

Compounds represented by structural formula **I** can be synthesized by the methods set forth in U.S. Patent 3,816,438. Compounds represented by structural formula II can be synthesized by the methods set forth in U.S. Patent 3,816,437.

Formation of the pyrazole ring is affected by heating compounds of the structure **IV** with hydrazine in an inert solvent. The reaction is preferably carried out for a period of 8 hours to 5 days at a temperature of 35 °C to 150°C, preferably at the reflux temperature of the system. Compound **V** is then dehydrogenated to compound **I** by exposure to silica or alumina in the presence of air or oxygen. Alternatively, a dehydrogenation agent, such as sulfur or palladium, or an oxidizing agent, such as manganese dioxide can be used. When a dehydrogenation agent or an oxidizing agent is used, it is preferable that an inert solvent is used, the reaction time is from 5 to 50 hours, and the reaction temperature is between 20° to 250°C. The following scheme is representative of this transformation.

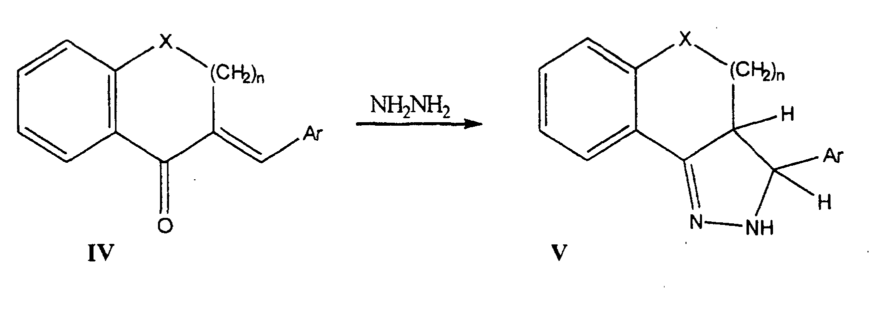

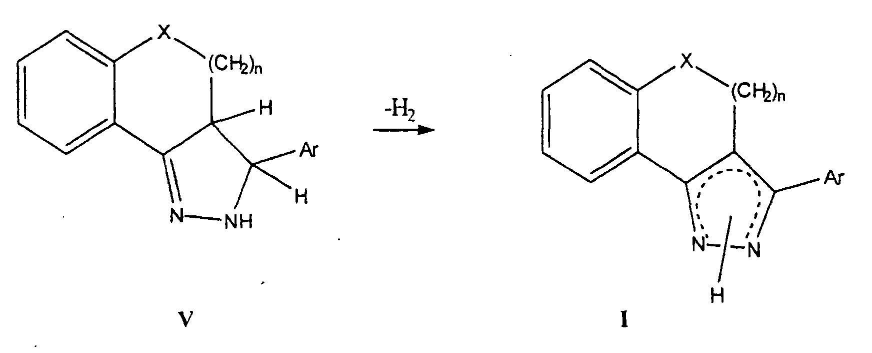

In a similar fashion, compound II can be formed by heating compound VI with hydrazine followed by dehydrogenation.

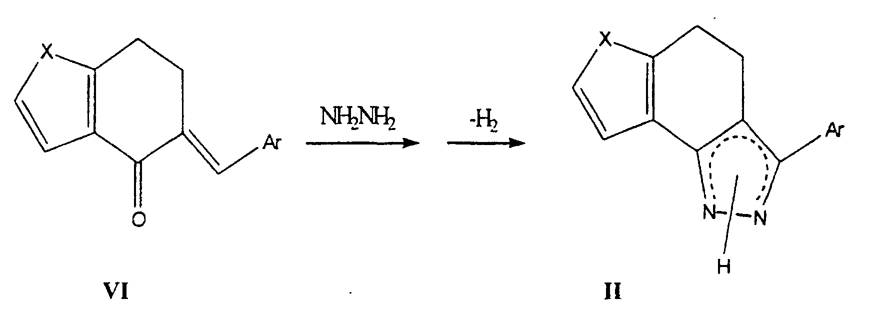

A second method of forming the pyrazole ring is by treating a compound represented by structural formula **VII** with hydrazine in an inert solvent. The reaction is carried out a temperature between 30° to 150°C, preferably at the reflux temperature of the reaction mixture. The following scheme is representative of this transformation.

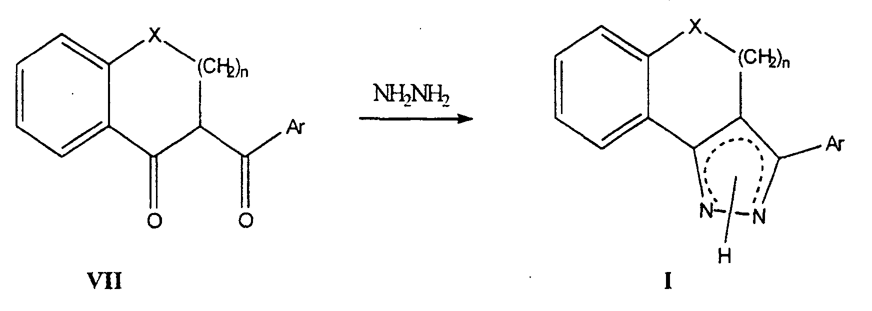

Again, in a similar fashion, compound **II** can be formed by treating compound **VIII** with hydrazine in an inert solvent.

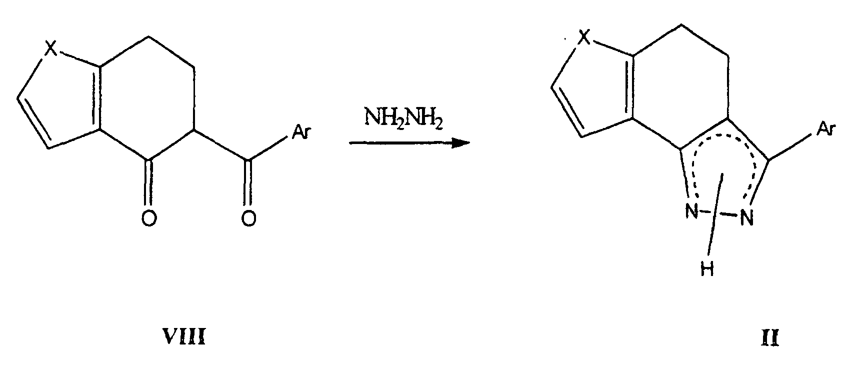

A third method of forming the pyrazole ring is by treating the epoxide represented by structural formula **IX** with hydrazine in an inert solvent in the presence of a catalytic amount of mineral acid, a strong organic acid or a Lewis acid. Preferred acids are p-toluenesulfonic acid and boron trifluoride. The reaction is carried out at a temperature between 35° to 200°C, preferably at the reflux temperature of the reaction mixture. The time course of the reaction is from 8 hours to 5 days. The following scheme is representative of this transformation.

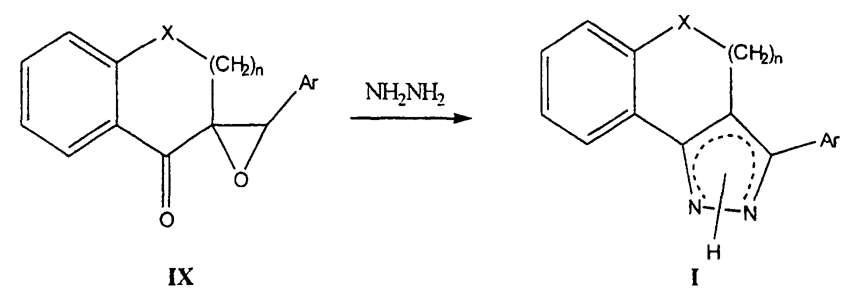

Again, in a similar fashion, compound **II** can be formed by treating compound **X** with hydrazine in an inert solvent with a catalytic amount of an acid.

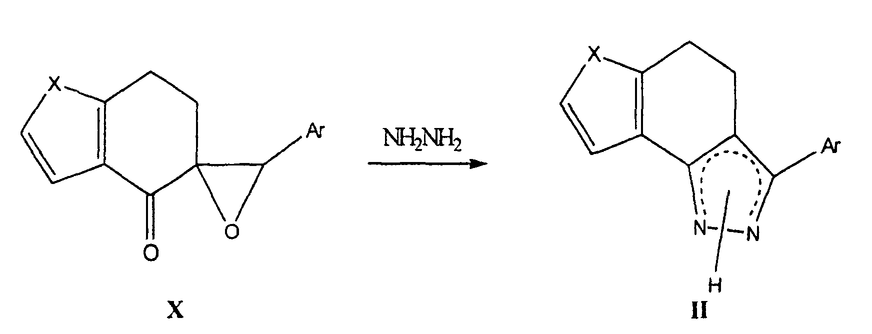

Compounds represented by structural formula **IV** are synthesized by treating a compound represented by formula **XI** with the appropriate aryl aldehyde **XII** in the presence of a catalytic amount of an acid or a base in an inert solvent. Suitable bases include sodium hydroxide, potassium hydroxide, triethylamine, or diethyl amine. Suitable acids include hydrochloric acid, sulfuric acid, phosphoric acid, acetic acid, toluenesulfonic acid and methyl sulfonic acid. The reaction temperature is from 15° to 100°C and the time course of the reaction is from 2 to 24 hours. The following scheme is representative of this transformation.

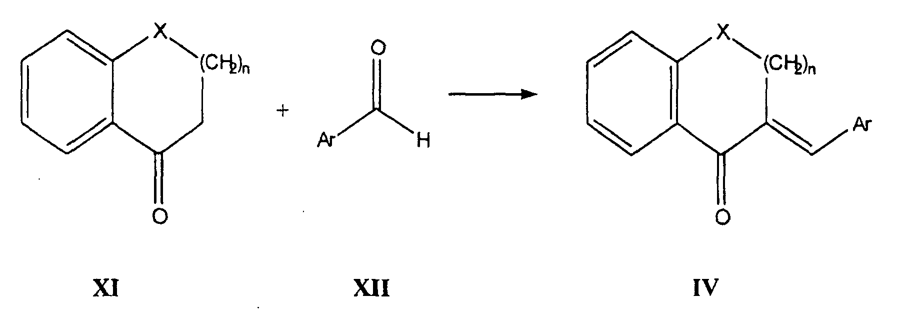

Similarly, compounds of structural formula **VI** can be synthesized from compounds of formula **XIII** by reacting these compounds with an appropriate aryl aldehyde in an inert solvent with a catalytic amount of an acid or base.

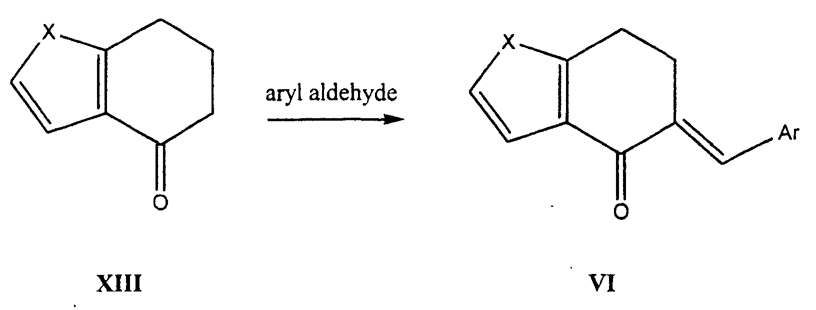

Compounds represented by structural formula **IX** are synthesized by treating a compound represented by formula **XIV** with an aryl aldehyde under basic conditions in an inert solvent under an inert atmosphere. Suitable bases include alkali or alkali earth metal hydroxides, alkali metal lower alkoxides, tertiary alphatic and aromatic amines, and tertiary cyclic amines such as pyridine. The reaction is carried out at 0° to 30°C for 1 to 5 hours. The following scheme is representative of this transformation. 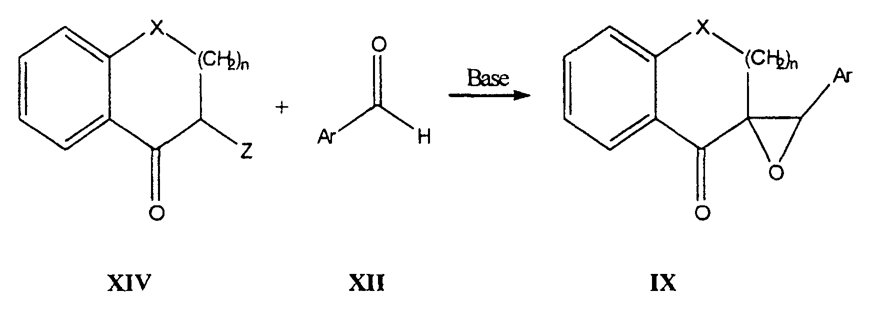 wherein Z is a leaving group, such as a halogen, tosylate, mesylate and the like.

Similarly, compounds of structural formula **X** can be synthesized from compounds of formula **XV** by reacting these compounds with an appropriate aryl aldehyde in an inert solvent under basic conditions.

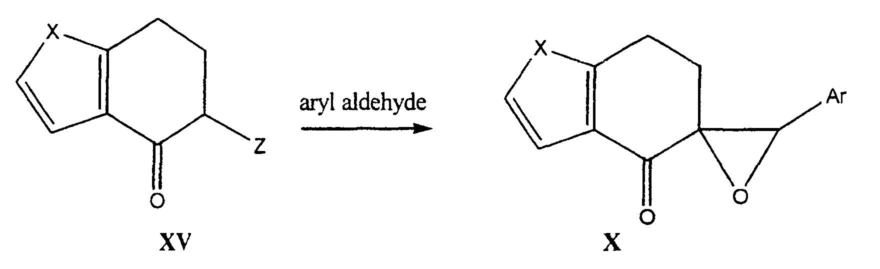

Compounds represented by structural formulas **XIV** and **XV** are synthesized by standard procedures from compounds represented by structural formulas **XI** and **XIII**. respectively. For example, a compound represented by structural formula **XI**, can be treated in an inert solvent with chlorine or bromine at temperatures ranging from room temperature to 50°C for a period of I to 12 hours to give a compound of formula **XIV**.

Compounds with structural formula **VII**, **VIII**, **XI**, and **XIII** are known and can be prepared by methods disclosed in the literature.

Specific examples of the above transformations can be found in U.S. Patent 3,816.438 or U.S. Patent 3,816,437.

### II. In Vitro Protein Kinase Assays

The following *in vitro* assays may be used to determine the level of activity and effect of the different compounds of the present invention on one or more of the protein kinases. Similar assays can be designed along the same lines for other protein kinases using techniques well known in the art.

### A. KDR Protein Production Using Baculovirus System:

The coding sequence for the KDR intra-cellular domain (aa789-1354) was generated through PCR using cDNAs isolated from HUVEC cells. A poly-His₆ sequence was introduced at the N-terminus of this protein as well. This fragment was cloned into transfection vector pVL1393 at the Xba 1 and Not 1 site. Recombinant baculovirus (BV) was generated through co-transfection using the BaculoGold Transfection reagent (PharMingen). Recombinant BV was plaque purified and verified through Western analysis. For protein production, SF-9 cells were grown in SF-900-II medium at 2 x 10⁶/ml, and were infected at 0.5 plaque forming units per cell (MOI). Cells were harvested at 48 hours post infection.

### B. Purification of KDR

SF-9 cells expressing (His)₆KDR(aa789-1354) were lysed by adding 50 ml of Triton X-100 lysis buffer (20 mM Tris, pH 8.0, 137 mM NaCl, 10% glycerol, 1% Triton X-100, 1mM PMSF, 10µg/ml aprotinin, 1 µg/ml leupeptin) to the cell pellet from 1L of cell culture. The lysate was centrifuged at 19,000 rpm in a Sorval SS-34 rotor for 30 min at 4°C. The cell lysate was applied to a 5 ml NiCl₂ chelating sepharose column, equilibrated with 50 mM HEPES, pH7.5, 0.3 M NaCl. KDR was eluted using the same buffer containing 0.25 M imidazole. Column fractions were analyzed using SDS-PAGE and an ELISA assay (below) which measures kinase activity. The purified KDR was exchanged into 25mM HEPES, pH7.5, 25mM NaCl, 5 mM DTT buffer and stored at -80°C.

### Human Tie-2 Kinase Production and Purification

The coding sequence for the human Tie-2 intra-cellular domain (aa775-1124) was generated through PCR using cDNAs isolated from human placenta as a template. A poly-His₆ sequence was introduced at the N-terminus and this construct was cloned into transfection vector pVL 1939 at the Xba 1 and Not 1 site. Recombinant BV was generated through co-transfection using the BaculoGold Transfection reagent (PharMingen). Recombinant BV was plaque purified and verified through Western analysis. For protein production, SF-9 insect cells were grown in SF-900-II medium at 2 x 10⁶/ml, and were infected at MOI of 0.5. Purification of the His-tagged kinase used in screening was analogous to that described for KDR.

### Human Flt-1 Tyrosine Kinase Production and Purification

The baculoviral expression vector pVL 1393 (Phar Mingen, Los Angeles, CA) was used. A nucleotide sequence encoding poly-His6 was placed 5' to the nucleotide region encoding the entire intracellular kinase domain of human Flt-1 (amino acids 786-1338). The nucleotide sequence encoding the kinase domain was generated through PCR using cDNA libraries isolated from HUVEC cells. The histidine residues enabled affinity purification of the protein as a manner analogous to that for KDR and ZAP70. SF-9 insect cells were infected at a 0.5 multiplicity and harvested 48 hours post infection.

### EGFR Tyrosine Kinase Source

EGFR was purchased from Sigma (Cat # E-3641; 500 units/50 µl) and the EGF ligand was acquired from Oncogene Research Products/Calbiochem (Cat # PF011-100).

### Expression of ZAP70

The baculoviral expression vector used was pVL1393. (Pharmingen, Los Angeles, Ca.) The nucleotide sequence encoding amino acids M(H)6 LVPR₉S was placed 5' to the region encoding the entirety of ZAP70 (amino acids 1-619). The nucleotide sequence encoding the ZAP70 coding region was generated through PCR using cDNA libraries isolated from Jurkat immortalized T-cells. The histidine residues enabled affinity purification of the protein (vide infra). The LVPR₉S bridge constitutes a recognition sequence for proteolytic cleavage by thrombin, enabling removal of the affinity tag from the enzyme. SF-9 insect cells were infected at a multiplicity of infection of 0.5 and harvested 48 hours post infection.

### Extraction and purification of ZAP70

SF-9 cells were lysed in a buffer consisting of 20 mM Tris, pH 8.0, 137 mM NaCl, 10% glycerol, 1% Triton X-100, 1 mM PMSF, 1 µg/ml leupeptin, 10 µg/ml aprotinin and I mM sodium orthovanadate. The soluble lysate was applied to a chelating sepharose HiTrap column (Pharmacia) equilibrated in 50 mM HEPES, pH 7.5, 0.3 M NaCl. Fusion protein was eluted with 250 mM imidazole. The enzyme was stored in buffer containing 50 mM HEPES, pH 7.5, 50 mM NaCl and 5 mM DTT.

### Lck source

Lck or truncated forms of Lck may be commercially obtained ( e.g. from Upstate Biotechnology Inc. (Saranac Lake, N.Y) and Santa Cruz Biotechnology Inc. (Santa Cruz, Ca.)) or purified from known natural or recombinant sources using conventional methods.

### Cdc2 source

The human recombinant enzyme and assay buffer may be obtained commercially (New England Biolabs, Beverly, MA. USA) or purified from known natural or recombinant sources using conventional methods.

### Cdc2 Assay

The protocol used was that provided with the purchased reagents with minor modifications. In brief, the reaction was carried out in a buffer consisting of 50mM Tris pH 7.5, 100mM NaCl, 1mM EGTA, 2mM DTT, 0.01% Brij, 5% DMSO and 10mM MgCl₂ (commercial buffer) supplemented with fresh 300 µM ATP (31 µCi/ml) and 30 µg/ml histone type IIIss final concentrations. A reaction volume of 80µL, containing units of enzyme, was run for 20 minutes at 25 degrees C in the presence or absence of inhibitor. The reaction was terminated by the addition of 120µL of 10% acetic acid. The substrate was separated from unincorporated label by spotting the mixture on phosphocellulose paper, followed by 3 washes of 5 minutes each with 75mM phosphoric acid. Counts were measured by a betacounter in the presence of liquid scintillant.

Certain compounds of this invention significantly inhibit cdc2 at concentrations below 50 µM.

### PKC kinase source

The catalytic subunit of PKC may be obtained commercially (Calbiochem).

### PKC kinase assay

A radioactive kinase assay was employed following a published procedure (Yasuda, I., Kirshimoto, A., Tanaka, S.. Tominaga, M., Sakurai, A., Nishizuka, Y. *Biochemical and Biophysical Research Communication* 3:166, 1220-1227 (1990)). Briefly, all reactions were performed in a kinase buffer consisting of 50 mM Tris-HCl pH7.5, 10mM MgCl₂, 2mM DTT, 1mM EGTA, 100 µM ATP, 8 µM peptide, 5% DMSO and ³³P ATP (8Ci/mM). Compound and enzyme were mixed in the reaction vessel and the reaction initiated by addition of the ATP and substrate mixture. Following termination of the reaction by the addition of 10 µL stop buffer (5 mM ATP in 75mM phosphoric acid), a portion of the mixture was spotted on phosphocellulose filters. The spotted samples were washed 3 times in 75 mM phosphoric acid at room temperature for 5 to 15 minutes. Incorporation of radiolabel was quantified by liquid scintillation counting.

### Erk2 enzyme source

The recombinant murine enzyme and assay buffer may be obtained commercially (New England Biolabs, Beverly MA. USA) or purified from known natural or recombinant sources using conventional methods.

### Erk2 enzyme assay

In brief, the reaction was carried out in a buffer consisting of 50 mM Tris pH 7.5. 1 mM EGTA, 2mM DTT, 0.01% Brij, 5% DMSO and 10 mM MgCl₂ (commercial buffer) supplemented with fresh 100 µM ATP (31 µCi/ml) and 30µM myelin basic protein under conditions recommended by the supplier. Reaction volumes and method of assaying incorporated radioactivity were as described for the PKC assay (vide supra).

### C. Enzyme Linked Immunosorbent Assay (ELISA)

Enzyme linked immunosorbent assays (ELISA) can be used to detect and measure the presence of tyrosine kinase activity. The ELISA can be conducted according to known protocols which are described in, for example. Voller, *et al.,* 1980, "Enzyme-Linked Immunosorbent Assay," In: *Manual of Clinical Immunology, 2d ed.,* edited by Rose and Friedman, pp 359-371 Am. Soc. of Microbiology, Washington, D.C.

The disclosed protocol can be adapted for determining activity with respect to a specific RTK. For example, preferred protocols for conducting the ELISA experiments for KDR is provided below. Adaptation of these protocols for determining a compounds activity for other members of the RTK family, as well as non-receptor tyrosine kinases, are well within the abilities of those in the art.

### KDR IN VITRO ELISA

The following procedure was used to assay the inhibitory effect of compounds of this invention on KDR tyrosine kinase activity:

### Buffers and Solutions:

- **PGT:**: Poly (Glu,Tyr) 4:1
Store powder at -20°C. Dissolve powder in phosphate buffered saline (PBS) for 50mg/ml solution. Store 1ml aliquots at -20°C. When making plates dilute to 250µg/ml in Gibco PBS.
- **Reaction Buffer:**: 100mM Hepes, 20mM MgCl₂, 4mM MnCl₂, 5mM DTT, 0.02%BSA, 200µM NaVO₄, ph 7.10
- **ATP:**: Store aliquots of 100mM at -20°C. Dilute to 20µM in water
- Washing Buffer:: PBS with 0.1 % Tween 20
- **Antibody Diluting Buffer:**: 0.1% bovine serum albumin (BSA) in PBS
- **TMB Substrate:**: mix TMB substrate and Peroxide solutions 9:1 just before use or use K-Blue Substrate from Neogen
- **Stop Solution:**: 1 M Phosphoric Acid

### Procedure

**1. Plate Preparation:**
   Dilute PGT stock (50mg/ml, frozen) in PBS to a 250µg/ml. Add 125µl per well of Coming modified flat bottom high affinity ELISA plates (Corning #25805-96). Add 125µl PBS to blank wells. Cover with sealing tape and incubate overnight 37°C. Wash 1x with 250µl washing buffer and dry for about 2hrs in 37°C dry incubator. Store coated plates in sealed bag at 4°C until used.
**2. Tyrosine Kinase Reaction:**
   - Prepare inhibitor solutions at a 4x concentration in 20% DMSO in water.
   - Prepare reaction buffer
   - Prepare enzyme solution so that desired units are in 50µl, e.g. for KDR make to 1 ng/µl for a total of 50ng per well in the reactions. Store on ice.
      - Make 4x ATP solution to 20µM from 100mM stock in water. Store on ice
   - Add 50µl of the enzyme solution per well
   - Add 25µl 4x inhibitor
   - Add 25µl 4x ATP for inhibitor assay
   - Incubate for 10 minutes at room temperature
   - Stop reaction by adding 50µl 0.05N HCl per well
   - Wash plate
   **Final Concentrations for Reaction:
   ATP: 5µM
   5% DMSO
**3. Antibody Binding**
   - Dilute 1mg/ml aliquot of PY20-HRP (Pierce) antibody to 50ng/ml in 0.1% BSA in PBS by a 2 step dilution (100x, then 200x)
   - Add 100µl Ab per well. Incubate 1 hr at room temp. Incubate 1hr at 4C.
   - Wash 4x plate
**4. Color reaction**
   - Prepare TMB substrate and add 100µl per well
   - Monitor OD at 650nm until 0.6 is reached
   - Stop with 1M Phosphoric acid. Shake on plate reader.
   - Read OD immediately at 450nm

Optimal incubation times and enzyme reaction conditions vary slightly with enzyme preparations and are determined empirically for each lot.

For Lck, the Reaction Buffer utilized was 100 mM MOPSO, pH 6.5, 4 mM MnCl₂, 20 mM MgCl₂, 5 mM DTT, 0.2% BSA, 200 mM NaVO₄ under the analogous assay conditions.

Compounds of formula I can have therapeutic utility in the treatment of diseases involving both identified, including those not mentioned herein, and as yet unidentified protein tyrosine kinases which are inhibited by compounds of formula I. All compounds exemplified herein significantly inhibit KDR kinase at concentrations of 50 micromolar or below. Some compounds of this invention also significantly inhibit other PTKs such as lck at concentrations of 50 micromolar or below.

### Results

The following inhibitory concentration of a representative compound was obtained:

| ELISA Assay | IC₅₀ for Compound **III** |
|---|---|
| KDR | 13.4 µM |
| Lck | > 30.0 µM |
| TIE2 | > 30.0 µM |
| zapfl | 30.3 µM |

These results demonstrate that compounds of the present invention have notable inhibitory activity for KDR tyrosine kinase as well as for other kinases.

### In Vitro Models for T-cell Activation

Upon activation by mitogen or antigen, T-cells are induced to secrete IL-2, a growth factor that supports their subsequent proliferative phase. Therefore, one may measure either production of IL-2 from or cell proliferation of, primary T-cells or appropriate T-cell lines as a surrogate for T-cell activation. Both of these assays are well described in the literature and their parameters well documented (in Current Protocols in Immunology, Vol 2, 7.10.1-7.11.2).

In brief, T-cells may be activated by co-culture with allogenic stimulator cells, a process termed the one-way mixed lymphophocyte reaction. Responder and stimulator peripheral blood mononuclear cells are purified by Ficoll-Hypaque gradient (Pharmacia) per directions of the manufacturer. Stimulator cells are mitotically inactivated by treatment with mitomycin C (Sigma) or gamma irradiation. Responder and stimulator cells are co-cultured at a ratio of two to one in the presence or absence of the test compound. Typically 10⁵ responders are mixed with 5 x 10⁴ stimulators and plated (200 µl volume) in a U bottom microtiter plate (Costar Scientific). The cells are cultured in RPMI 1640 supplemented with either heat inactivated fetal bovine serum (Hyclone Laboratories), or pooled human AB serum from male donors, 5 x 10⁻⁵ M 2mercaptoethanol and 0.5% DMSO, The cultures are pulsed with 0.5 µCi of ³H thymidine (Amersham) one day prior to harvest (typically day three). The cultures are harvested (Betaplate harvester, Wallac) and isotope uptake assessed by liquid scintillation (Betaplate, Wallac).

The same culture system may be used for assessing T-cell activation by measurement of IL-2 production. Eighteen to twenty-four hours after culture initiation, the supernatants are removed and the IL-2 concentration is measured by ELISA (R and D Systems) following the directions of the manufacturer.

### In-vivo Models of T-Cell Activation

The *in vivo* efficacy of compounds can be tested in animal models known to directly measure T-cell activation or for which T-cells have been proven the effectors. T-cells can be activated *in vivo* by ligation of the constant portion of the T-cell receptor with a monoclonal anti-CD3 antibody (Ab). In this model, BALB/c mice are given 10µg of anti-CD3 Ab intraperitoneally two hours prior to exsanguination. Animals to receive a test drug are pre-treated with a single dose of the compound one hour prior to anti-CD3 Ab administration. Serum levels of the proinflammatory cytokines interferon-γ (IFN- γ) and tumor necrosis factor-α(TNF-α), indicators of T-cell activation, are measured by ELISA. A similar model employs *in vivo* T-cell priming with a specific antigen such as keyhole limpet hemocyanin (KLH) followed by a *secondary in vitro* challenge of draining lymph node cells with the same antigen. As previously, measurement of cytokine production is used to assess the activation state of the cultured cells. Briefly, C57BL/6 mice are immunized subcutaneously with 100 µg KLH emulsified in complete Freund's adjuvant (CFA) on day zero. Animals are pre-treated with the compound one day prior to immunization and subsequently on days one, two and three post immunization. Draining lymph nodes are harvested on day 4 and their cells cultured at 6 x 10⁶ per ml in tissue culture medium (RPMI 1640 supplemented with heat inactivated fetal bovine serum (Hyclone Laboratories), 5 x 10⁻⁵ M 2-mercaptoethanol and 0.5% DMSO) for both twenty-four and forty-eight hours. Culture supernatants are then assessed for the autocrine T-cell growth factor Interleukin-2 (IL-2) and/or IFN-γ levels by ELISA.

Lead compounds can also be tested in animal models of human disease. These are exemplified by experimental auto-immune encephalomyelitis (EAE) and collagen-induced arthritis (CIA). EAE models which mimic aspects of human multiple sclerosis have been described in both rats and mice (reviewed FASEB J. 5:2560-2566, 1991; murine model: Lab. Invest. 4(3):278, 1981; rodent model:J. Immunol 146(4):1163-8, 1991 ). Briefly, mice or rats are immunized with an emulsion of myelin basic protein (MBP), or neurogenic peptide derivatives thereof, and CFA. Acute disease can be induced with the addition of bacterial toxins such as *bordetella pertussis.* Relapsing/remitting disease is induced by adoptive transfer of T-cells from MBP/ peptide immunized animals.

CIA may be induced in DBA/1 mice by immunization with type II collagen (J. Immunol:142(7):2237-2243). Mice will develop signs of arthritis as early as ten days following antigen challenge and may be scored for as long as ninety days after immunization. In both the EAE and CIA models, a compound may be administered either prophylactically or at the time of disease onset. Efficacious drugs should reduce severity and/or incidence.

Certain compounds of this invention which inhibit one or more angiogenic receptor PTK, and/or a protein kinase such as lck involved in mediating inflammatory responses can reduce the severity and incidence of arthritis in these models.

Compounds can also be tested in mouse allograft models, either skin (reviewed in Ann. Rev. Immunol., 10:333-58, 1992; Transplantation: 57(12): 1701-17D6, 1994) or heart (Am.J.Anat.:113:273, 1963). Briefly, full thickness skin grafts are transplanted from C57BL/6 mice to BALB/c mice. The grafts are examined daily, beginning at day six, for evidence of rejection. In the mouse neonatal heart transplant model, neonatal hearts are ectopically transplanted from C57BL/6 mice into the ear pinnae of adult CBA/J mice. Hearts start to beat four to seven days post transplantation and rejection may be assessed visually using a dissecting microscope to look for cessation of beating.

### Cellular Receptor PTK Assays

The following cellular assay was used to determine the level of activity and effect of the different compounds of the present invention on KDR/VEGFR2. Similar receptor PTK assays employing a specific ligand stimulus can be designed along the same lines for other tyrosine kinases using techniques well known in the art.

VEGF-Induced KDR Phosphorylation in Human Umbilical Vein Endothelial Cells (HUVEC) as Measured by Western Blots.
1. HUVEC cells (from pooled donors) were purchased from Clonetics (San Diego, CA) and cultured according to the manufacturer directions. Only early passages (3-8) were used for this assay. Cells were cultured in 100 mm dishes (Falcon for tissue culture; Becton Dickinson; Plymouth, England) using complete EBM media (Clonetics).
2. For evaluating a compound's inhibitory activity, cells were trypsinized and seeded at 0.5-1.0 x 10⁵ cells/well in each well of 6-well cluster plates (Costar; Cambridge, MA).
3. 3-4 days after seeding, plates were 90-100% confluent. Medium was removed from all the wells, cells were rinsed with 5-10ml of PBS and incubated 18-24h with 5ml of EBM base media with no supplements added (i.e., serum starvation).
4. Serial dilutions of inhibitors were added in 1ml of EBM media (25µM, 5µM, or 1µM final concentration to cells and incubated for one hour at 37°C. Human recombinant VEGF₁₆₅ ( R & D Systems) was then added to all the wells in 2 ml of EBM medium at a final concentration of 50ng/ml and incubated at 37°C for 10 minutes. Control cells untreated or treated with VEGF only were used to assess background phosphorylation and phosphorylation induction by VEGF.

All wells were then rinsed with 5-10ml of cold PBS containing 1 mM Sodium Orthovanadate (Sigma) and cells were lysed and scraped in 200µl of RIPA buffer (50mM Tris-HC1) pH7, 150mM NaCl, 1% NP-40, 0.25% sodium deoxycholate, 1mM EDTA) containing protease inhibitors (PMSF 1mM, aprotinin 1µg/ml, pepstatin 1µg/ml, leupeptin 1µg/ml, Na vanadate 1mM, Na fluoride 1mM) and 1µg/ml of Dnase (all chemicals from Sigma Chemical Company, St Louis, MO). The lysate was spun at 14,000 rpm for 30min, to eliminate nuclei.

Equal amounts of proteins were then precipitated by addition of cold (-20°C) Ethanol (2 volumes) for a minimum of 1 hour or a maximum of overnight. Pellets were reconstituted in Laemli sample buffer containing 5% β-mercaptoethanol (BioRad; Hercules. CA) and boiled for 5min. The proteins were resolved by polyacrylamide gel electrophoresis (6%, 1.5mm Novex, San Deigo, CA) and transferred onto a nitrocellulose membrane using the Novex system. After blocking with bovine serum albumin (3%), the proteins were probed overnight with anti-KDR polyclonal antibody (C20, Santa Cruz Biotechnology; Santa Cruz, CA) or with anti-phosphotyrosine monoclonal antibody (4G10, Upstate Biotechnology, Lake Placid, NY) at 4°C. After washing and incubating for 1 hour with HRP-conjugated F(ab)₂ of goat anti-rabbit or goat-anti-mouse IgG the bands were visualized using the emission chemiluminescience (ECL) system (Amersham Life Sciences, Arlington Height, IL).

Certain examples of the present invention significantly inhibit cellular VEGF-induced KDR tyrosine kinase phosphorylation at concentrations of less than 50 µM.

### In Vivo Uterine Edema Model

This assay measures the capacity of compounds to inhibit the acute increase in uterine weight in mice which occurs in the first few hours following estrogen stimulation. This early onset of uterine weight increase is known to be due to edema caused by increased permeability of uterine vasculature. Cullinan-Bove and Koss *(Endocrinology* (1993), *133*:829-837) demonstrated a close temporal relationship of estrogen-stimulated uterine edema with increased expression of VEGF mRNA in the uterus. These results have been confirmed by the use of neutralizing monoclonal antibody to VEGF which significantly reduced the acute increase in uterine weight following estrogen stimulation (WO 97/42187). Hence, this system can serve as a model for *in vivo* inhibition of VEGF signalling and the associated hyperpermeability and edema.
Materials: All hormones were purchased from Sigma (St. Louis, MO) or Cal Biochem (La Jolla, CA) as lyophilized powders and prepared according to supplier instructions.
Vehicle components (DMSO, Cremaphor EL) were purchased from Sigma (St. Louis, MO).
Mice (Balb/c, 8-12 weeks old) were purchased from Taconic (Germantown, NY) and housed in a pathogen-free animal facility in accordance with institutional Animal Care and Use Committee Guidelines.

### Method:

Day 1: Balb/c mice were given an intraperitoneal (i.p.) injection of 12.5 units of pregnant mare's serum gonadotropin (PMSG).
Day 3: Mice received 15 units of human chorionic gonadotropin (hCG) i.p.
Day 4: Mice were randomized and divided into groups of 5-10. Test compounds were administered by i.p., i.v. or p.o. routes depending on solubility and vehicle at doses ranging from 1-100 mg/kg. Vehicle control group received vehicle only and two groups were left untreated.

Thirty minutes later, experimental, vehicle and 1 of the untreated groups were given an i.p. injection of 17 β-estradiol (500 µg/kg). After 2-3 hours, the animals were sacrificed by CO₂ inhalation. Following a midline incision, each uterus was isolated and removed by cutting just below the cervix and at the junctions of the uterus and oviducts. Fat and connective tissue were removed with care not to disturb the integrity of the uterus prior to weighing. Mean weights of treated groups were compared to untreated or vehicle treated groups. Significance was determined by Student's t-test. Non-stimulated control group was used to monitor estradiol response.

Results demonstrate that certain compounds of the present invention inhibit the formation of edema when administered systemically by various routes.

Certain compounds of this invention which are inhibitors of angiogenic receptor tyrosine kinases can also be shown active in a Matrigel implant model of neovascularization. The Matrigel neovascularization model involves the formation of new blood vessels within a clear "marble" of extracellular matrix implanted subcutaneously which is induced by the presence of proangiogenic factor producing tumor cells (for examples see: Passaniti, A., et al, Lab. Investig. (1992), 67(4), 519-528; Anat. Rec. (1997), 249(1), 63-73; Int. J. Cancer (1995), 63(5), 694-701; Vasc. Biol. (1995), 15(11), 1857-6). The model preferably runs over 3-4days and endpoints include macroscopic visual/image scoring of neovascularization, microscopic microvessel density determinations, and hemoglobin quantitation (Drabkin method) following removal of the implant versus controls from animals untreated with inhibitors. The model may alternatively employ bFGF or HGF as the stimulus.

Certain compounds of this invention which inhibit one or more oncogenic, protooncogenic, or proliferation-dependent protein kinases, or angiogenic receptor PTK also inhibit the growth of primary murine, rat or human xenograft tumors in mice, or inhibit metastasis in murine models.

### EQUIVALENTS

While this invention has been particularly shown and described with references to preferred embodiments thereof, it will be understood by those skilled in the art that various changes in form and details may be made therein without departing from the scope of the invention as defined by the appended claims. Those skilled in the art will recognize or be able to ascertain using no more than routine experimentation. many equivalents to the specific embodiments of the invention described specifically herein. Such equivalents are intended to be encompassed in the scope of the claims.

## Claims

1. Use of a compound represented by either of the formulas: 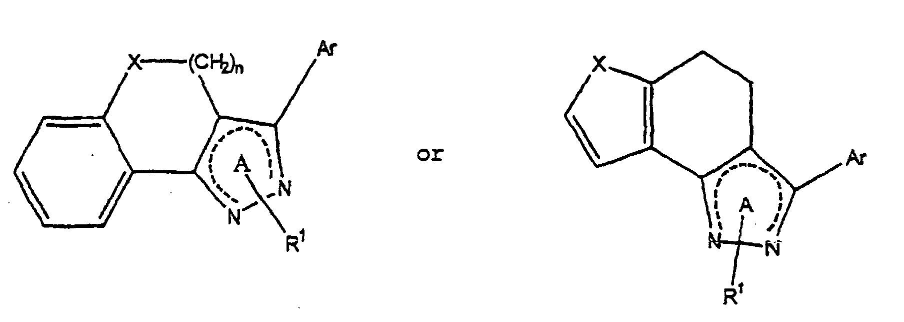 for the manufacture of a medicament in sufficient concentration for inhibiting one or more protein kinase activity wherein:
X is oxygen or sulfur;
ring A represents the tautomeric structures 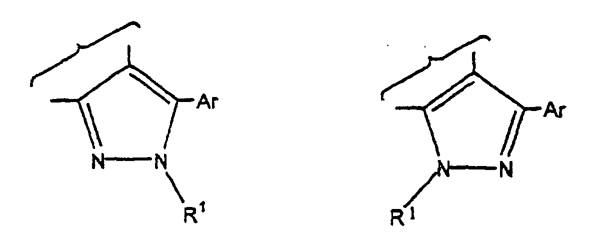
n is 1 or 2;
Ar is 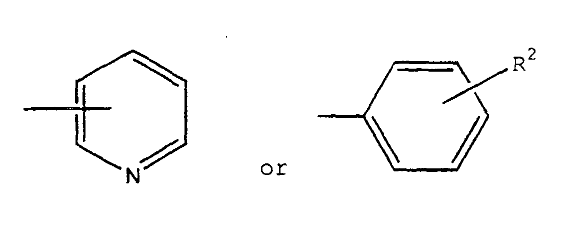 and
R¹ is hydrogen, methyl, -COR³, or -SO₂R³;
R² represents a halogen having an atomic weight of about 19 to 36; and
R³ is -CH₃ or 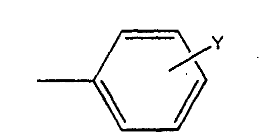 wherein Y is hydrogen, chlorine or -CH₃

2. The use of Claim 1, wherein said compound is in a stereoisomeric form or is a mixture of stereoisomers.

3. The use of Claim 1, wherein said compound is in a mixture with one or more other said compounds.

4. The use of Claim 1, wherein said protein kinase is either a receptor tyrosine kinase or a non-receptor tyrosine kinase.

5. The use of Claim 4, wherein said tyrosine kinase is selected from the group consisting of KDR, flt-1,TIE-2, Lck, Src, Fyn and yes.

6. The use of Claim 1, wherein the activity of said protein kinase affects angiogenesis.

7. The use of Claim 6, wherein the inhibition of said protein kinase is anti-angiogenic.

8. The use of Claim 7, wherein said inhibition of said protein kinase inhibits the progression of a disease state selected from the group consisting of cancer, arthritis, atherosclerosis, psoriasis, hemangioma, myocardial angiogenesis, coronary and cerebral collaterals, ischemic limb angiogenesis, corneal disease, rubeosis, neovascular glaucoma, macular degeneration, wound healing, peptic ulcer *Helicobacter* related diseases, fractures, diabetic retinopathy, and cat scratch fever.

9. The use of Claim 1, wherein the activity of said protein kinase affects vascular hyperpermeability or the production of edema.

10. The use of Claim 9, wherein the inhibition of said protein kinase is antiedematous.

11. The use of Claim 10, wherein the inhibition of said protein kinase inhibits the progression of a disease state selected from the group consisting of bums, chronic lung disease, stroke, polyps, psoriasis, allergic inflammation, macular degeneration, diabetic retinopathy, ovarian hyperstimulation syndrome and brain tumor-associated cerebral edema.

12. The use of Claim 1, wherein said protein kinase is a serine kinase.

13. The use of Claim 1, wherein said protein kinase is a threonine kinase.

14. The use of Claim 1, wherein said compound is

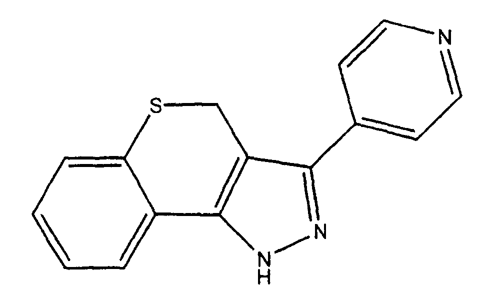

## Patentansprüche

1. Verwendung einer Verbindung der Formel: 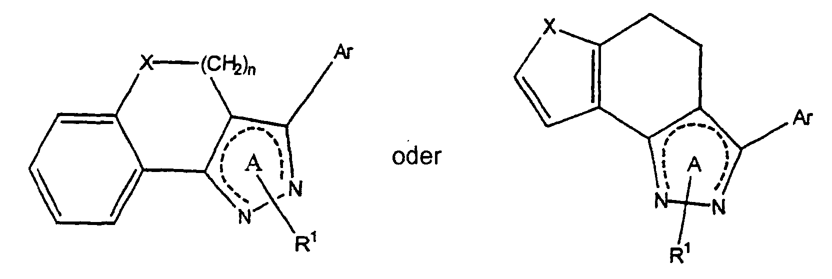 bei der Herstellung eines Medikaments in ausreichender Konzentration zur Inhibierung einer oder mehrerer Proteinkinase-Aktivitäten, wobei
X Sauerstoff oder Schwefel ist;
Ring A für die tautomeren Strukturen 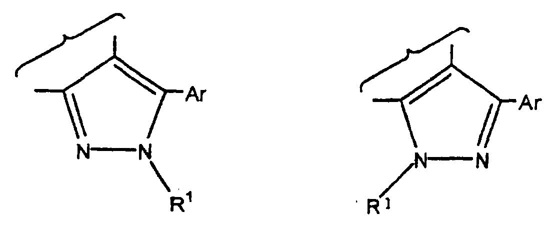 steht;
n 1 oder 2 ist;
Ar 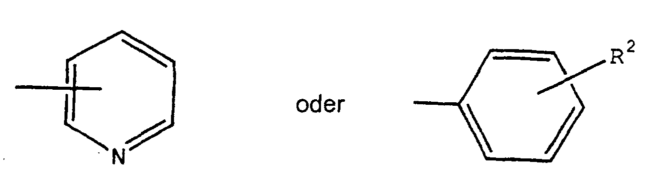 ist; und
R¹ für Wasserstoff, Methyl, -COR³ oder -SO₂R³ steht;
R² für Halogen mit einem Atomgewicht von etwa 19 bis 36 steht; und
R³ für -CH₃ oder 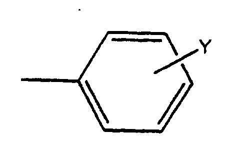 steht, worin Y für Wasserstoff, Chlor oder -CH₃ steht.

2. Verwendung nach Anspruch 1, wobei die Verbindung in stereoisomerer Form oder als Gemisch von Stereoisomeren vorliegt.

3. Verwendung nach Anspruch 1, wobei die Verbindung als Gemisch mit einer oder mehreren anderen von den Verbindungen vorliegt.

4. Verwendung nach Anspruch 1, wobei die Proteinkinase entweder eine Tyrosinkinase vom Rezeptortyp oder eine Tyrosinkinase vom Nichtrezeptortyp ist.

5. Verwendung nach Anspruch 4, wobei die Tyrosinkinase ausgewählt ist unter KDR, flt-1, TIE-2, Lck, Src, Fyn und yes.

6. Verwendung nach Anspruch 1, wobei die Aktivität der Proteinkinase die Angiogenese beeinflusst.

7. Verwendung nach Anspruch 6, wobei die Inhibition der Proteinkinase antiangiogen ist.

8. Verwendung nach Anspruch 7, wobei die Inhibition der Proteinkinase die Progression eines Erkrankungszustandes inhibiert, der ausgewählt ist unter Krebs, Arthritis, Atherosklerose, Psoriasis, Hämangiome, myokardiale Angiogenese, koronare und zerebrale Kollaterale, Angiogenese ischämischer Gliedmaßen, kornealer Erkrankung, Rubeose, neovaskulären Glaukomen, Makuladegeneration, Wundheilung, Magengeschwür, mit *Helicobacter* in Zusammenhang stehenden Erkrankungen, Frakturen, diabetischer Retinopathie und Katzenkratzfieber.

9. Verwendung nach Anspruch 1, wobei die Aktivität der Proteinkinase die vaskuläre Hyperpermeabilität oder die Ödemproduktion beeinflusst.

10. Verwendung nach Anspruch 9, wobei die Inhibition der Proteinkinase anti-ödematös ist.

11. Verwendung nach Anspruch 10, wobei die Inhibition der Proteinkinase die Progression eines Erkrankungszustandes inhibiert, der ausgewählt ist unter Verbrennungen, chronischer Lungenerkrankung, Gehirnschlag, Polypen, Psoriasis, allergischer Entzündung, Makuladegeneration, diabetischer Retinopathie, ovarialem Hyperstimulationssyndrom und mit einem Gehirntumor in Zusammenhang stehenden zerebralen Ödemen.

12. Verwendung nach Anspruch 1, wobei die Proteinkinase eine Serinkinase ist.

13. Verwendung nach Anspruch 1, wobei die Proteinkinase eine Threoninkinase ist.

14. Verwendung nach Anspruch 1, wobei die Verbindung 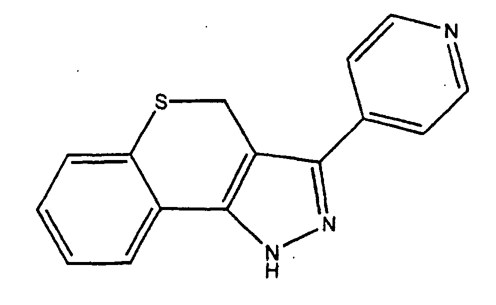 ist.

## Revendications

1. Utilisation d'un composé représenté par l'une ou l'autre des formules 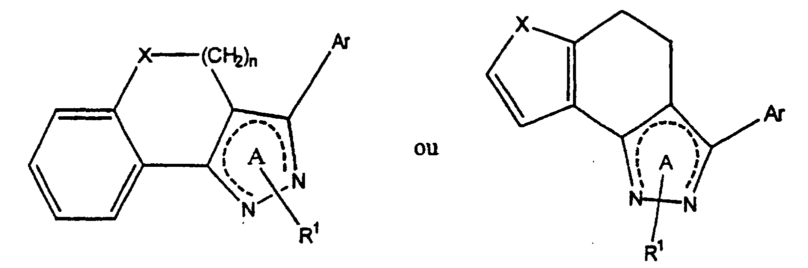 pour la fabrication d'un médicament en concentration suffisante pour l'inhibition d'une ou plusieurs activités de protéine-kinase, où
X représente l'oxygène ou le soufre;
le cycle A représente les structures tautomères 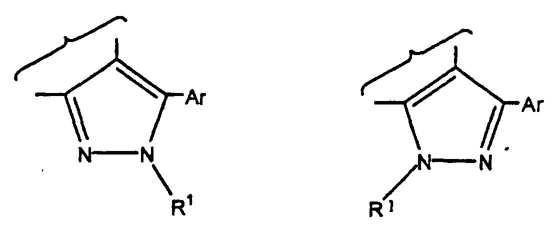 n est 1 ou 2;
Ar représente 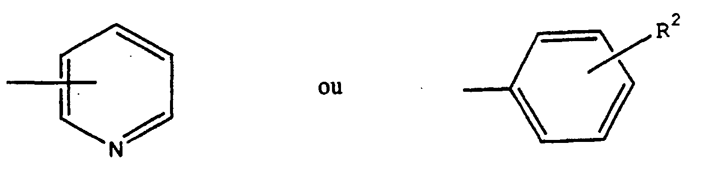 et
R¹ est un atome d'hydrogène ou un groupe méthyle, -COR³ ou -SO₂R³;
R² représente un halogène ayant une masse atomique d'environ 19 à 36; et
R³ est un groupe -CH₃ ou 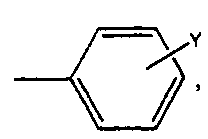 où Y est un atome d'hydrogène ou de chlore ou un groupe -CH₃.

2. Utilisation selon la revendication 1, dans laquelle ledit composé est sous une forme stéréoisomère ou est un mélange de stéréoisomères.

3. Utilisation selon la revendication 1, dans laquelle ledit composé se trouve dans un mélange avec un ou plusieurs autres de ces composés.

4. Utilisation selon la revendication 1, dans laquelle ladite protéine-kinase est soit une tyrosine-kinase de récepteur, soit une tyrosine-kinase de non récepteur.

5. Utilisation selon la revendication 4, dans laquelle ladite tyrosine-kinase est choisie dans le groupe constitué par KDR, flt-1, TIE-2, Lck, Src, Fyn et yes.

6. Utilisation selon la revendication 1, dans laquelle l'activité de ladite protéine-kinase a un effet sur l'angiogenèse.

7. Utilisation selon la revendication 6, dans laquelle l'inhibition de ladite protéine-kinase est antiangiogénique.

8. Utilisation selon la revendication 7, dans laquelle ladite inhibition de ladite protéine-kinase inhibe l'évolution d'un état pathologique choisi dans le groupe constitué par le cancer, l'arthrite, l'athérosclérose, le psoriasis, l'hémangiome, l'angiogenèse myocardique, les anastomoses coronariennes et cérébrales, l'angiogenèse ischémique des membres, la maladie cornéenne, la rubéose, le glaucome néovasculaire, la dégénérescence maculaire, la cicatrisation, les ulcères peptiques associés à *Helicobacter,* les fractures, la rétinopathie diabétique et la maladie des griffes du chat.

9. Utilisation selon la revendication 1, dans laquelle l'activité de ladite protéine-kinase a un effet sur l'hyperperméabilité vasculaire ou la production d'oedèmes.

10. Utilisation selon la revendication 9, dans laquelle l'inhibition de ladite protéine-kinase est antioedémateuse.

11. Utilisation selon la revendication 10, dans laquelle l'inhibition de ladite protéine-kinase inhibe l'évolution d'un état pathologique choisi dans le groupe constitué par les brûlures, des maladies pulmonaires chroniques, l'ictus, les polypes, le psoriasis, les inflammations allergiques, la dégénérescence maculaire, la rétinopathie diabétique, le syndrome d'hyperstimulation ovarienne et l'oedème cérébral associé à une tumeur cérébrale.

12. Utilisation selon la revendication 1, dans laquelle ladite protéine-kinase est une sérine-kinase.

13. Utilisation selon la revendication 1, dans laquelle ladite protéine-kinase est une thréonine-kinase.

14. Utilisation selon la revendication 1, dans laquelle ledit composé est
